# EUROPÄISCHE PATENTSCHRIFT

(11) **EP 0 904 053 B1**
(45) Veröffentlichungstag und Bekanntmachung des Hinweises auf die Patenterteilung: **16.10.2002**
(21) Anmeldenummer: 97922978.8
(22) Anmeldetag: 06.05.1997
(51) Int. Cl.: A61K 7/50, A61K 7/06

(54) **HAARBEHANDLUNGSMITTEL**
HAIR TREATMENT AGENTS
AGENTS DE TRAITEMENT CAPILLAIRE

(30) Priorität: 15.05.1996 DE 19619645
(43) Veröffentlichungstag der Anmeldung: 31.03.1999
(73) Patentinhaber: Cognis Deutschland GmbH & Co. KG, 40589 Düsseldorf (DE)
(72) Erfinder: ANSMANN, Achim, D-40699 Erkrath (DE)
(86) Internationale Anmeldenummer: EP9702303
(87) Internationale Veröffentlichungsnummer: WO97042935

(56) Entgegenhaltungen:
- EP-A- 0 398 177
- EP-A- 0 531 650
- EP-A- 0 643 961
- DE-A- 4 435 383
- FR-A- 2 702 145

## Beschreibung

### Gebiet der Erfindung

Die Erfindung betrifft Haarbehandlungsmitteln mit einem definierten Gehalt an Zuckertensiden, ausgewählten Siliconverbindungen und Perlglanzwachsen.

### Stand der Technik

Seit Beginn der 60er Jahre haben Siliconverbindungen als Bestandteile kosmetischer Zubereitungen zunehmend an Bedeutung gewonnen, da sie das Haut- und Haargefühl dieser Mittel schon in kleinen Mengen verbessern, chemisch inert und mit praktisch allen kosmetischen Inhaltsstoffen kompatibel und schließlich aus dermatologischer Sicht unbedenklich sind. Zuckertenside vom Typ der Alkylglucoside oder Fettsäure-N-alkylglucamide stellen ebenfalls wegen ihrer ausgezeichneten anwendungstechnischen Eigenschaften in Kombination mit besonderer hautkosmetischer Verträglichkeit bevorzugte Tenside für die Herstellung beispielsweise von Haarshampoos und Handgeschirrspülmitteln dar, denen man aus ästhetischen Gründen häufig Stoffe zusetzt, die den Formulierungen infolge von Lichtstreuung an winzigen Kristallen einen Perlglanz verleiht. Eine Übersicht zu modernen perlglänzenden Formulierungen findet sich von A.Ansmann et al. in **Parf.Kosm. 75**, **578 (1994)**.

Perlglanzkonzentrate, die acylierte Ethylenglycole zusammen mit Alkylglucosiden enthalten, sind beispielsweise aus den beiden Europäischen Patentschriften **EP-B1 0376083** und **EP-B1 0570398** (Henkel) sowie die Internationale Patentanmeldung **WO 95/13863** (SEPPIC) bekannt.

Zusammensetzungen, die Alkyloligoglykoside, bei Raumtemperatur flüssige Silicone und Perlglanzwachse enthalten, beschreiben die beiden Europäischen Patentschriften **EP-B1 0337354** und **EP-B1 0398177** (Kao). So werden beispielsweise in der letztgenannten Schrift in den Beispielen 2 und 3 Shampoozusammensetzungen offenbart, die jeweils 20 Gew.-% Alkylglucosid, 3 Gew.-% flüssige Siloxane und 2 Gew.-% Perlglanzwachse, nämlich Ethylenglycoldistearat, enthalten. In der Praxis zeigt sich, daß Formulierung dieser Art nicht stabil sind, inhomogen werden und eindicken. Zwar wird die Trübung durch den Gehalt an Perlglanzwachsen kaschiert, die Zubereitungen sind jedoch hinsichtlich ihres Erscheinungsbildes nicht brillant, sondern wirken stumpf, was vom Verbraucher oft mit Begriffen wie "verdorben" oder "überlagert" gleichgesetzt wird. Es ist sofort klar, daß ein solches Produkt, obschon von seinen Eigenschaften her einwandfrei, auf wenig Kaufinteresse stößt.

Aus dem Europäischen Patent **EP-B1 0557399** (L'Oreal) sind ähnliche Zubereitungen bekannt, die neben Alkylglucosiden und Perlglanzwachsen ebenfalls Silicone enthalten, bei denen es sich jedoch nicht um bei Raumtemperatur feste, harzartige Stoffe handelt, die für die Herstellung beispielsweise von Haarshampoos höchstens eingeschränkt in Betracht kommen, da sie leicht zu einem Verkleben der Haare führen können. Kosmetische Zubereitungen mit kurzkettigen Alkylglucosiden und hydroxymodifizierten Polysiloxanen sind schließlich aus der Französischen Patentschrift **FR-B 2542997** (L'Oreal) bekannt; diese Mittel enthalten jedoch keine Perlglanzwachse. Weiterhin sei auf die US-Patentschriften **US 4,559,227** (Dow) sowie **US 4,704,252** und **US 4,741,855** (Procter & Gamble) verwiesen, die Perlglanzshampoos mit Siliconen offenbaren.

Demzufolge hat die Aufgabe der Erfindung darin bestanden, Haarbehandlungsmittel mit Perlglanzeffekt auf Basis der genannten Zuckertenside und flüssigen Siliconverbindungen zur Verfügung zu stellen, die ausreichend lagerstabil sind und einen brillanten, feinteiligen Perlglanz zeigen.

### Beschreibung der Erfindung

Gegenstand der Erfindung sind Haarbehandlungsmittel, enthaltend
(a) 1 bis 30 Gew.-% Zuckertenside,
(b) 1 bis 10 Gew.-% bei Raumtemperatur flüssigen Siliconverbindungen und
(c) weniger als 2 Gew.-% Perlglanzwachse,
mit der Maßgabe, daß sich die Mittel mit Wasser und üblichen Zusatzstoffen zu 100 Gew.-% ergänzen

In umfangreichen Untersuchungen hat die Anmelderin gefunden, daß sich überraschenderweise Inhomogenitäten bei den Haarshampoos des Stands der Technik zuverlässig vermeiden lassen, wenn man den Gehalt an Perlglanzwachsen in den Mitteln auf unter 2, vorzugsweise auf 0,1 bis 1,5 und insbesondere auf 0,5 bis 1,0 Gew.-% einstellt. Die resultierenden Zubereitungen sind auch bei Lagerung unter schwankenden Temperaturbedingungen lagerstabil, d.h. es finden keine Austrübungen statt, die Viskosität bleibt konstant und der Perlglanz weist die gewünschte Brillanz und Feinteiligkeit auf.

### Alkyl- und/oder Alkenyloligoglykoside

Alkyl- und Alkenyloligoglykoside stellen bekannte nichtionische Tenside dar, die der Formel **(I)** folgen,

**R**^{**1**}**O-[G]**_{**p**} **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1 0301298** und **WO 90/03977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkylund/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3, die in Mengen von 1 bis 30 und vorzugsweise 5 bis 20 Gew.-% - bezogen auf die Mittel - eingesetzt werden.

### Fettsäure-N-alkylpolyhydroxyalkylamide

Fettsäure-N-alkylpolyhydroxyalkylamide stellen nichtionische Tenside dar, die der Formel **(II)** folgen, 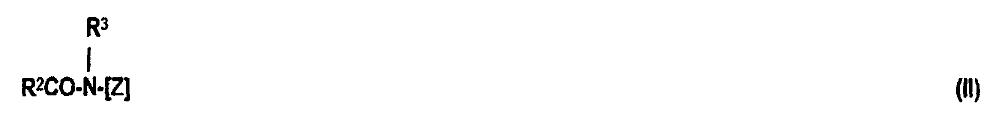 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens.Surf.Deterg. 25, 8 (1988).**

Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(III)** wiedergegeben werden:

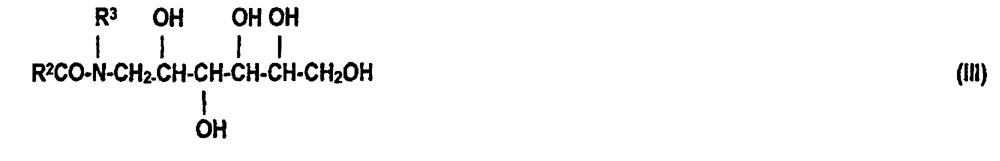

Vorzugsweise werden als Fettsäure-N-alkylpolyhydroxyalkylamide Glucamide der Formel **(III)** eingesetzt, in der R³ für eine Alkylgruppe steht und R²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkyl-glucamide der Formel **(III)**, die durch reduktive Aminierung von Glucose mit Methylamin und anschließende Acylierung mit Laurinsäure oder C_{12/14}-Kokosfettsäure bzw. einem entsprechenden Derivat erhalten werden, Weiterhin können sich die Polyhydroxyalkytamide auch von Maltose und Palatinose ableiten. Vorzugsweise werden die Amide in Mengen von 1 bis 30 und insbesondere 5 bis 25 Gew.-% eingesetzt. Es sind auch Mischungen mit den vorgenannten Alkyl- undloder Alkenyloligoglykosiden im Gewichtsverhältnis 10 : 90 bis 90 : 10, vorzugsweise 25 : 75 bis 75 : 25 und insbesondere 40 : 60 bis 60 : 40 möglich.

Auch die Verwendung der Fettsäure-N-alkylpolyhydroxyalkylamide ist Gegenstand einer Vielzahl von Veröffentlichungen. Aus der Europäischen Patentanmeldung **EP-A1 0285768** (Hüls) ist beispielsweise ihr Einsatz als Verdickungsmittel bekannt. In der Französischen Offenlegungsschrift **FR-A 1580491** (Henkel) werden wäßrige Detergensgemische auf Basis von Sulfaten und/oder Sulfonaten, Niotensiden und gegebenenfalls Seifen beschrieben, die Fettsäure-N-alkylglucamide als Schaumregulatoren enthalten. Mischungen von kurz- und längerkettigen Glucamiden werden in der Deutschen Patentschrift **DE-C1 44 00 632** (Henkel) beschrieben. In den Deutschen Offenlegungsschriften **DE-A1 4336958** und **DE-A1 4309567** (Henkel) wird ferner über den Einsatz von Glucamiden mit längeren Alkylresten als Pseudoceramide in Hautpflegemitteln sowie über Kombinationen von Glucamiden mit Proteinhydrolysaten und kationischen Tensiden in Haarpflegeprodukten berichtet.

### Flüssige Siliconverbindungen

Die Siliconverbindungen, die im Sinne der Erfindung als Komponente (b) in Betracht kommen, sind bei Raumtemperatur, also im Bereich von 18 bis 25°C, flüssig und stellen vorzugsweise Dimethylpolysiloxane oder Methylphenylpolysiloxane dar. Eine Übersicht hierzu findet sich beispielsweise von K.Schnurrbusch in **Seifen-Fette-Öle-Wachse 100, 173 (1974)**. Weitere geeignete Stoffe sind aminomodifizierte Silicone, beispielsweise vom Amodimethicone-Typ sowie offenkettige oder cyclische Siliconverbindungen, die gegebenenfalls durch Fettsäure-, Alkohol-, Polyether-, Epoxy-, Fluor- oder Alkylgruppen substituiert sind. Eine Übersicht zu den in Frage kommenden Stoffen findet sich in der EP-B1 0 398 177, deren Lehre zur Natur der Siliconverbindungen hiermit ausdrücklich eingeschlossen wird. Die Silicone können in Mengen von 1 bis 10, vorzugsweise 2 bis 5 Gew.-% - bezogen auf die Mittel - eingesetzt werden.

### Perlglanzwachse

Als Perlglanzwachse kommen vorzugsweise Acylierungsprodukte von Glycolen und deren Eigenkondensationsprodukten in Frage, die der Formel **(IV)** folgen, 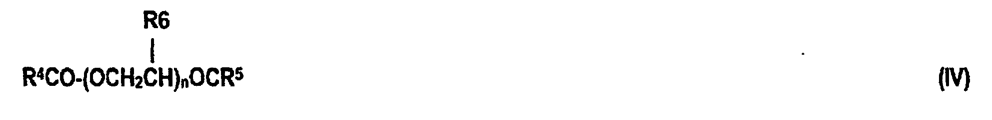 in der R⁴CO und R⁵CO unabhängig von einander für gesättigte oder ungesättigte Acylreste mit 8 bis 22, vorzugsweise 12 bis 18 Kohlenstoffatomen, R⁶ für Wasserstoff oder eine Methylgruppe und n für Zahlen von 1 bis 5 steht. Vorzugsweise kommen für diesen Zweck Acylierungsprodukte des Ethylenglycols mit Kokosfettsäure oder Stearinsäure, wie insbesondere Ethylenglycoldistearat in Betracht.

Eine weitere Gruppe von Perlglanzwachsen stellen Fettsäurepartialester dar, die der Formel **(V)** folgen, 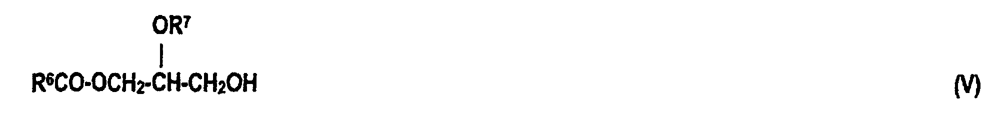 in der R⁶CO für einen gesättigten oder ungesättigten Acylrest mit 6 bis 22, vorzugsweise 12 bis 18 Kohlenstoffatomen und R⁷ für Wasserstoff oder einen Acylrest R⁶CO steht. Typische Beispiele sind Mono- und Diglyceride sowie deren technischen Gemische, die herstellungsbedingt noch geringe Anteile an Trifettsäureestem enthalten können. Gegenüber den acylierten Glycolen weisen sie den Vorteil auf, daß sie keine Ethylenoxideinheiten enthalten, was von verschiedenen Herstellern kosmetischer Zubereitungen bevorzugt wird. In einer bevorzugten Ausführungsform der Erfindung werden daher beispielsweise Mono/Diglyceridgemische eingesetzt, die sich von der Laurinsäure, der Kokosfettsäure, der Palmitinsäure, der Öl-säure und insbesondere der Stearinsäure ableiten. Neben den oben genannten klassischen Perlglanzwachse können auch anorganische Perlglanzpigmente wie beispielsweise vom Typ der gecoateten Montmorillonite eingesetzt werden.

### Gewerbliche Anwendbarkeit

Die erfindungsgemäßen Haarbehandlungsmittel können weitere, mit den anderen Inhaltsstoffen kompatible **Tenside** enthalten. Typische Beispiele sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Ethercarbonsäuren, Alkylamidobetaine und/oder vorzugsweise pflanzliche Proteinfettsäurehydrolysate bzw. deren Kondensate mit Fettsäuren. Sie können ferner als weitere Hilfs- und Zusatzstoffe Ölkörper, Emulgatoren, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Adsorber, Farb- und Duftstoffe enthalten.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, Ester von C₆-C₂₂-Fettalkohoten und/oder Guerbetalkoholen mit aromatischen Carbonsäuren, insbesondere Benzoesäure, pflanzliche Öle, verzweigte primäre Alkohole, substituierte Cyclohexane, Guerbetcarbonate, Dialkylether und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(b1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlenstoffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{12/22}-Fettsäuren, Ricinotsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b9) Trialkylphosphate;
(b10) Wollwachsalkohole;
(b11) Polysiloxan-Polyalkyl-Polyether-Copolymere bzw. entsprechende Derivate;
(b12) Mischester aus Pentaerythrit, Fettsäuren, Citronensäure und Fettalkohol gemäß **DE-PS 11 65 574** und/oder Mischester von Fettsäuren mit 6 bis 22 Kohlenstoffatomen, Methylglucose und Polyolen, vorzugsweise Glycerin sowie
(b13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmonound -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen, quatemierte Kollagenpolypeptide wie beispielsweise Lauryldimonium hydroxypropyl hydrolyzed collagen (Lamequat®L, Grünau GmbH), quatemierte Weizenpolypeptide, Polyethylenimin, kationische Siliconpolymere wie z.B. Amidomethicone oder Dow Coming, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Zur Verbesserung des Fließverhaltens können ferner **Hydrotrope** wie beispielsweise Ethanol, Isopropylalkohol, Propylenglycol oder Glucose eingesetzt werden. Als **Konservierungsmittel** eignen sich beispielsweise Phenoxyethanol, Formaldehydlösung, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

1 bis 2 g Perlglanzwachs wurden aufgeschmolzen, mit 15 g Zuckertensid, 5 g Siliconverbindung, den weiteren tensidischen Inhaltsstoffen versetzt und mit Wasser auf 100 g ergänzt. Nach Homogenisierung wurden die Shampooformulierungen 1 Woche bei 40°C gelagert. Die Viskosität der Produkte wurde nach der Brookfieldmethode (23°C, Spindel 5, 10 Upm) ermittelt, die Feinteiligkeit der Perlglanzkristalle unter dem Mikroskop visuell auf einer Skala von 1 = sehr feine Kristalle bis 5 = grobe Kristalle beurteilt. Die Beurteilung des Perlglanzes erfolgte ebenfalls auf einer Skala von 1 = brillant bis 5 = stumpf; die Trübung wurde visuell bestimmt und mit (+) = trüb oder (-) = trübungsfrei beurteilt. Die Ergebnisse sind in Tabelle 1 zusammengefaßt (Prozentangaben als Gew.-%). Die Rezepturen R1 bis R5 sind erfindungsgemäß, die Rezepturen R6 bis R 10 dienen zum Vergleich.

**Tabelle 1**

| **Perlglanzshampoos** | | | | | | | | | | |
|---|---|---|---|---|---|---|---|---|---|---|
| **Komponenten** | **R1** | **R2** | **R3** | **R4** | **R5** | **R6** | **R7** | **R8** | **R9** | **R10** |
| Kokosalkyloligoglucosid | 15,0 | 15,0 | 15,0 | 15,0 | - | 15,0 | 15,0 | 15,0 | 15,0 | - |
| Laurinsäure-N-methylglucamid | - | - | - | - | 15,0 | - | - | - | - | 15,0 |
| Kokosfettalkohol+2EO-sulfat-Na-Salz | 10.0 | 10,0 | 10,0 | 10,0 | 10,0 | 10,0 | 10,0 | 10,0 | 10,0 | 10,0 |
| Dimethylpolysiloxan | 5,0 | 5,0 | - | - | 5,0 | 5,0 | 5,0 | - | - | 5,0 |
| Amodimethicone | - | - | 5,0 | 5,0 | - | - | - | 5,0 | 5,0 | - |
| Cocoamidopropyl Betaine | 5,0 | 5,0 | 5,0 | 5,0 | 5,0 | 5,0 | 5,0 | 5,0 | 5,0 | 5,0 |
| Esterquat* | 1,5 | 1,5 | 1,5 | 1,5 | 1,5 | 1,5 | 1,5 | 1,5 | 1,5 | 1,5 |
| Ethylenglycoldistearat | 1,0 | - | 1,5 | - | - | 2,0 | - | 2,0 | - | - |
| Laurinsäuremonoglycerid | - | 0,5 | - | 1,5 | 1,0 | - | 2,0 | - | 2,0 | 2,0 |
| Wasser | ad 100 | | | | | | | | | |
| ***Viskosität [mPas]*** | 30 | 30 | 26 | 30 | 40 | >40 | >40 | >40 | >40 | >40 |
| ***Feinteiligkeit*** | 1 | 1 | 1 | 1 | 2 | 3 | 3 | 3 | 3 4 | |
| ***Perlglanz*** | 1 | 2 | 1 | 1 | 1 | 3 | 3 | 3 | 3 | 5 |
| ***Trübung*** | - | - | - | - | - | + | + | + | + | + |

| | | | | | | | | | | |
|---|---|---|---|---|---|---|---|---|---|---|
| *) Dehyquart® F 75 (Henkel KGaA, Düsseldorf/FRG) | | | | | | | | | | |

## Patentansprüche

1. Haarbehandlungsmittel, enthaltend
(a) 1 bis 30 Gew.-% Zuckertenside,
(b) 1 bis 10 Gew.-% bei Raumtemperatur flüssige Siliconverbindungen und
(c) weniger als 2 Gew.-% Perlglanzwachse,
mit der Maßgabe, daß sich die Mittel mit Wasser und üblichen Zusatzstoffen zu 100 Gew.-% ergänzen.

2. Mittel nach Anspruch 1, **dadurch gekennzeichnet, daß** sie als Zuckertenside Alkyl- und Alkenyloligoglykoside der Formel **(I)** enthalten,
**R**^{**1**}**O-[G]**_{**p**} **(I)**
in der R¹ für einen Alkyl- undloder Alkenylrest mit 4 bis 22 Kohlenstoffatomen, G für einen Zuckerrest mit 5 oder 6 Kohlenstoffatomen und p für Zahlen von 1 bis 10 steht.

3. Mittel nach den Ansprüchen 1 und 2, **dadurch gekennzeichnet, daß** sie als Zuckertenside Fettsäure-N-alkylpolyhydroxyalkylamide der Formel **(II)** enthalten, 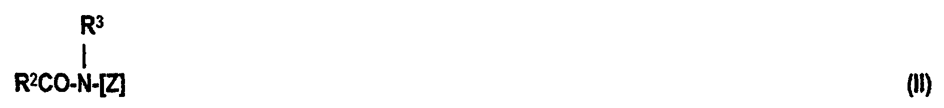 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

4. Mittel nach den Ansprüchen 1 bis 3, **dadurch gekennzeichnet, daß** sie flüssige Siliconverbindungen enthalten, die ausgewählt sind aus der Gruppe, die gebildet wird von Dimethylpolysiloxanen, Methylphenylpolysiloxanen sowie offenkettigen oder cyclischen Derivaten, die gegebenenfalls durch Amino-, Fettsäure-, Alkohol-, Polyether-, Epoxy-, Fluor- oder Alkylgruppen substituiert sind.

5. Mittel nach den Ansprüchen 1 bis 4, **dadurch gekennzeichnet, daß** sie als Perlgianzwachse Acylierungsprodukte von Glycolen und deren Eigenkondensationsprodukten der Formel **(IV)** enthalten, 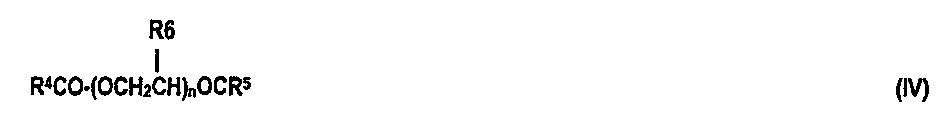 in der R⁴CO und R⁵CO unabhängig von einander für gesättigte oder ungesättigte Acylreste mit 8 bis 22 Kohlenstoffatomen, R⁶ für Wasserstoff oder eine Methylgruppe und n für Zahlen von 1 bis 5 steht.

6. Mittel nach den Ansprüchen 1 bis 5, **dadurch gekennzeichnet, daß** sie als Perlglanzwachse Fettsäurepartialester der Formel **(V)** enthalten, 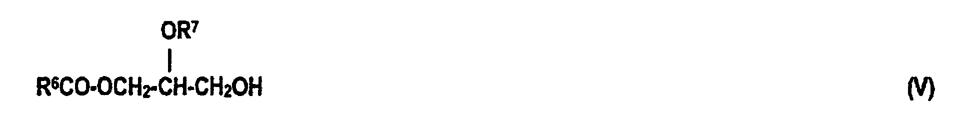 in der R⁶CO für einen gesättigten oder ungesättigten Acylrest mit 6 bis 22 Kohlenstoffatomen und R⁷ für Wasserstoff oder einen Acylrest R⁶CO steht.

## Claims

1. Hair-treatment formulations containing
(a) 1 to 30% by weight of sugar surfactants,
(b) 1 to 10% by weight of silicone compounds liquid at room temperature and
(c) less than 2% by weight of pearlescing waxes,
with the proviso that the formulations are made up to 100% by weight with water and typical additives.

2. Formulations as claimed in claim 1, **characterized in that** they contain alkyl and alkenyl oligoglycosides corresponding to formula **(I):**
**R**^{**1**}**O-[G]**_{**p**} **(I)**
where R¹ is an alkyl and/or alkenyl radical containing 4 to 22 carbon atoms, G is a sugar unit containing 5 or 6 carbon atoms and p is a number of 1 to 10, as sugar surfactants.

3. Formulations as claimed in claims 1 and 2, **characterized in that** they contain fatty acid N-alkyl polyhydroxyalkylamides corresponding to formula **(II):** 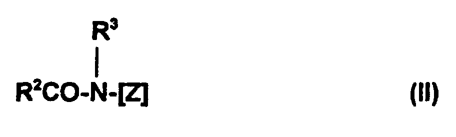 where R²CO is an aliphatic acyl radical containing 6 to 22 carbon atoms, R³ is hydrogen, an alkyl or hydroxyalkyl radical containing 1 to 4 carbon atoms and [Z] is a linear or branched polyhydroxyalkyl radical containing 3 to 12 carbon atoms and 3 to 10 hydroxyl groups,
as sugar surfactants.

4. Formulations as claimed in claims 1 to 3, **characterized in that** they contain liquid silicone compounds selected from the group consisting of dimethyl polysiloxanes, methylphenyl polysiloxanes and open-chain or cyclic derivatives optionally substituted by amino, fatty acid, alcohol, polyether, epoxy, fluorine or alkyl groups.

5. Formulations as claimed in claims 1 to 4, **characterized in that** they contain acylation products of glycols and self-condensation products thereof corresponding to formula **(IV)**: 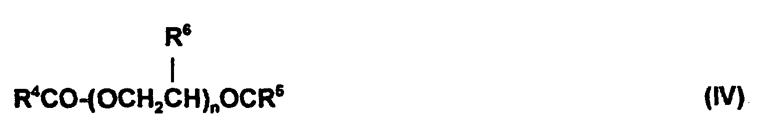 in which R⁴CO and R⁵CO independently of one another represent saturated or unsaturated acyl radicals containing 8 to 22 carbon atoms, R⁶ is hydrogen or a methyl group and n is a number of 1 to 5,
as pearlescing waxes.

6. Formulations as claimed in claims 1 to 5, **characterized in that** they contain fatty acid partial esters corresponding to formula **(V)**: 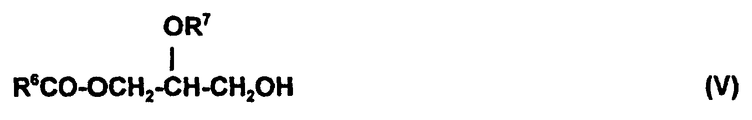 in which R⁶CO is a saturated or unsaturated acyl radical containing 6 to 22 carbon atoms and R⁷ is hydrogen or an acyl radical R⁶CO,
as pearlescing waxes.

## Revendications

1. Agents de traitement capillaire contenant :
a) de 1 à 30 % en poids d'agents tensioactifs dérivé de sucre,
b) de 1 à 10 % en poids de composés de silicone liquides à température ambiante, et
c) moins de 2 % en poids de cires à éclat nacré,
avec la restriction que les agents se complètent avec l'eau et les additifs usuels à 100 % en poids.

2. Agents selon la revendication 1,
**caractérisés en ce qu'**
ils contiennent comme agents tensioactifs dérivés de sucre, des alkyl et des alkényloligoglycosides de formule I,
R¹O-[G]ₚ (I)
dans laquelle R¹ représente un reste alkyle et/ou alkényle ayant de 4 à 22 atomes de carbone, G représente un reste de sucre ayant 5 ou 6 atomes de carbone et p représente des nombres allant de 1 à 10.

3. Agents selon l'une quelconque des revendications 1 et 2,
**caractérisés en ce qu'**
ils contiennent comme agents tensioactifs dérivés de sucres des N-alkylpolyhydroxyalkylamines d'acide gras de formule (II), 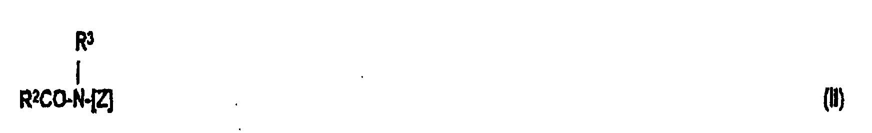 dans laquelle
R²CO représente un reste acyle aliphatique ayant de 6 à 22 atomes de carbone, R³ représente de l'hydrogène, un reste alkyle ou hydroxyélkyle ayant de 1 à 4 atomes de carbone, et [Z] représente un reste polyhydroxyalkyle linéaire ou ramifié ayant de 3 à 12 atomes de carbone et de 3 à 10 groupes hydroxyle.

4. Agents selon l'une quelconque des revendications 1 à 3,
**caractérisés en ce qu'**
ils contiennent des composés de silicone liquides qui sont choisis dans le groupe fermé des polydiméthylsiloxanes, des méthylphénylpolysiloxanes ainsi que des dérivés à chaîne ouverte ou cycliques qui sont éventuellement substitués par des groupes amine, un acide gras, un alcool, un polyéther, un époxy, un fluor, ou alkyle.

5. Agents selon l'une quelconque des revendications 1 à 4,
**caractérisés en ce qu'**
ils contiennent comme cires à éclat nacré des produits d'acylation de glycols et leurs produits d'autocondensation de formule (IV), 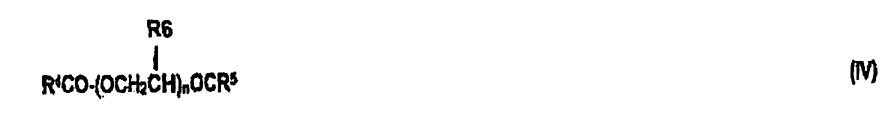 dans laquelle R⁴CO et R⁵CO indépendamment l'un de l'autre, représentent des restes acyle saturés ou non saturés ayant de 8 à 22 de préférence de 12 à 18 atomes de carbone, R⁶ représente de l'hydrogène, ou un groupe méthyle et n représente des nombres allant de 1 à 5.

6. Agents selon l'une quelconque des revendications 1 à 5,
**caractérisés en ce qu'**
ils contiennent comme cires à éclat nacré des esters partiels d'acide gras de formule (V), 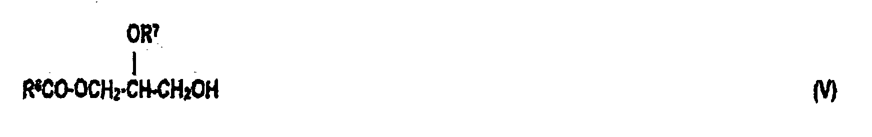 dans laquelle R⁶CO représente un reste acyle saturé ou non saturé ayant de 6 à 22, de préférence de 12 à 18 atomes de carbone et R⁷ représente de l'hydrogène ou un reste acyle R⁶CO.
